(19) Europäisches Patentamt
European Patent Office
Office européen des brevets

(11) **EP 3 430 980 A1**

(12) **EUROPEAN PATENT APPLICATION**

(43) Date of publication:
**23.01.2019  Bulletin 2019/04**

(51) Int Cl.:
**A61B 5/00** (2006.01)          **A61B 5/024** (2006.01)
**A61B 5/0205** (2006.01)      **A61B 5/1455** (2006.01)

(21) Application number: **17182481.6**

(22) Date of filing: **21.07.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **HILGERS, Achim
  5656 AE Eindhoven (NL)**
• **MUEHLSTEFF, Jens
  5656 AE Eindhoven (NL)**
• **SCHMITT, Lars
  5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **AN APPARATUS FOR MEASURING A PHYSIOLOGICAL PARAMETER USING A WEARABLE SENSOR**

(57)      A sensor apparatus comprises a housing, a support structure carried by the housing and a sensor for sensing movement of a contact portion of the support structure. The sensor is adapted to move relative to the housing. The apparatus further comprises a positioning system for adjusting the in-plane position of the support structure and sensor relative to the housing. This sensor design makes use of a support structure to decouple the sensor from the housing, so that there is a low inertial mass of the sensor, and hence a high sensitivity. In addition, a positioning system enables the signal to noise ratio to be maximized for a given placement of the housing, by enabling adjustment of the sensor position within the housing.

FIG. 11

EP 3 430 980 A1

**Description**

FIELD OF THE INVENTION

This invention relates to sensors for measuring a physiological parameter of a subject, wherein the sensor can be worn by a user. Examples of wearable sensors include a photoplethysmographic (PPG) sensor, an electrocardiogram sensor, an ultrasound sensor, a heart rate sensor, and a skin monitoring sensor.

BACKGROUND OF THE INVENTION

It is becoming increasingly popular to monitor a physiological parameter of a subject using an apparatus including a wearable device. Such devices are convenient to use as they offer increased freedom of movement for the user whilst a physiological parameter is being monitored. In this way, it is possible to measure physiological parameters in a variety of circumstances, for example at different levels of physical exertion of the subject.

The physiological parameter sensor may be mounted to the user differently in different applications. The position of the mounted physiological parameter sensor relative to the subject may vary according to the parameter to be measured, the type of physiological parameter sensor, and/or the circumstances in which physiological sensing takes place.

In many cases, the physiological parameter sensor should be in contact with the user's body. One issue with respect to wearable sensors is ensuring that the contact between the physiological parameter sensor and the user's body is maintained and the contact pressure is maintained at a comfortable level.

One example of a sensor which has become particularly common is a wrist-worn heart rate monitor. More recently, a patch for wearing over the carotid artery in the neck has also been proposed, where a stronger pulse is present. A worn heart rate sensor enables a user to track some of their vital signals in a simple unobtrusive way. These sensors typically use either an optical PPG sensor which measures a blood volume pulse or else they use a bio-impedance sensor. Capacitive methods have also been investigated to sense heart rate.

Heart rate monitoring is of increasing interest. In Europe, there are between 350,000 and 700,000 out of hospital sudden cardiac arrest (SCA) events every year which are frequently witnessed by (untrained) lay people. Cardiopulmonary resuscitation (CPR) is the key life-saving intervention recommended by international guidelines for victims of SCA. Adult CPR involves the delivery of chest compressions (CCs) at a depth of 5 to 6 cm and at a rate of 100 cpm, in combination with artificial ventilation (i.e., rescue breaths) to maintain the circulatory flow and oxygen supply to the vital organs in the body until spontaneous circulation returns. Guidelines recommend that during adult CPR, chest compressions and ventilations are delivered over a 2 minute interval at a ratio of 30 compressions to 2 ventilations, followed by a 10 second pause to perform a pulse check. If no pulse is detected then this cycle is repeated.

For successful resuscitation, it is vital that CPR is performed as soon as possible. Currently, international guidelines recommend that the cardiac arrest situation should be recognized within 5 to 10 seconds by performing a breathing check. With every minute that passes prior to initiating CPR, the probability of saving a victim's life decreases by 10% and after 10 minutes there is almost no chance of successful resuscitation. Moreover, poor CC performance (i.e., insufficient depth, rate or duration of CCs) by lay rescuers is one of the main factors contributing to the low reported post-CPR survival rate for victims of out of hospital cardiac arrest.

There is therefore a need for a solution that can assist rescuers to rapidly identify a cardiac arrest situation requiring CPR, i.e., by recognition that a victim has no pulse (and is not breathing). In addition, once CPR is initiated by lay rescuers there is also a need for a solution to assist in the detection of the return of spontaneous circulation (ROSC), so that they can determine when to stop CPR.

Several methods for pulse detection are taught in the prior art including the use of analysis of transthoracic impedance and ECG, Doppler shift due to electromagnetic waves transmitted from a defibrillation pad as well as the optical characteristics of light transmitted into a patient to ascertain physiological signals. None of this prior art is suitable for portable pulse detection by lay rescuers, nor are these solutions suitable for ubiquitous access during a sudden cardiac arrest or emergency event.

There is one commercially available pulse detection solution for lay rescuers called the "CPR Check" (CardiAid, California, USA). This device detects both the pulse and respiration at the suprasternal notch by using a resonant inductive-capacitive (L-C) sensor. The CPR Check is designed to output a decision regarding a victim's pulse and breathing status after 10 seconds using a color coded display output. However, use of the device still needs experience or training.

There remains a need for a solution which reliably and effectively assists lay rescuers in pulse assessment for initiation or cessation of CPR during a SCA emergency situation. A solution needs to be placement-insensitive, easy to apply and should provide a quantifiable pulse signal including robustness versus motion artefacts.

PPG sensors provide one possible measurement approach. PPG based sensors have the disadvantage that they have relatively high power consumption, as a result of the required LED. Thus, these sensors suffer from a short battery life. Capacitive and bio-impedance based methods (as well as some PPG sensors) also require good contact of the sensor with the skin which presents further design difficulties. Some sensors are also susceptible to motion artifacts, making them inaccurate in cases of severe or particular motions of the subject.

More recently, heart rate and respiration rate sensors have been proposed using an accelerometer or gyroscope, which aims to measure subtle motions caused by arterial expansion and the beating of the heart. These may be described as motion-based methods. One such approach is for example described in "Biowatch: Estimation of heart and breathing rates from wrist motions", J. Hernandez, D. McDuff, R.W. Picard, 9th International Conference on Pervasive Computing Technologies for Healthcare (PervasiveHealth) 2015, pp. 169-176.

A combination of sensor modalities may be used to improve the accuracy of results, for example using any combination of accelerometers, gyroscopes, and PPG sensors. The detection of individual blood pulses enables heart rate measurements, but also heart rate variability and heart rhythm measurements.

As with some other sensing modalities, motion sensing for sensing arterial (or other blood vessel) movements requires contact with the skin, and the sensitivity needs to be high. In particular, pulse detection from the carotid artery or other places from the body has the problem of very low amplitudes based on measuring the dilatation using motion sensors. Typical skin doming for healthy subjects due to the passing of a pulse at the carotid artery is in the order of 0.1 mm - 1 mm. The skin doming is caused by the dilatation of the carotid artery which is located about 1 cm - 1.5 cm below the skin surface. The dilatation is transferred via soft tissue to the skin. Therefore, any pressure or a sensing element with high mass inertia at the skin surface e.g. an accelerometer will dampen the effect significantly.

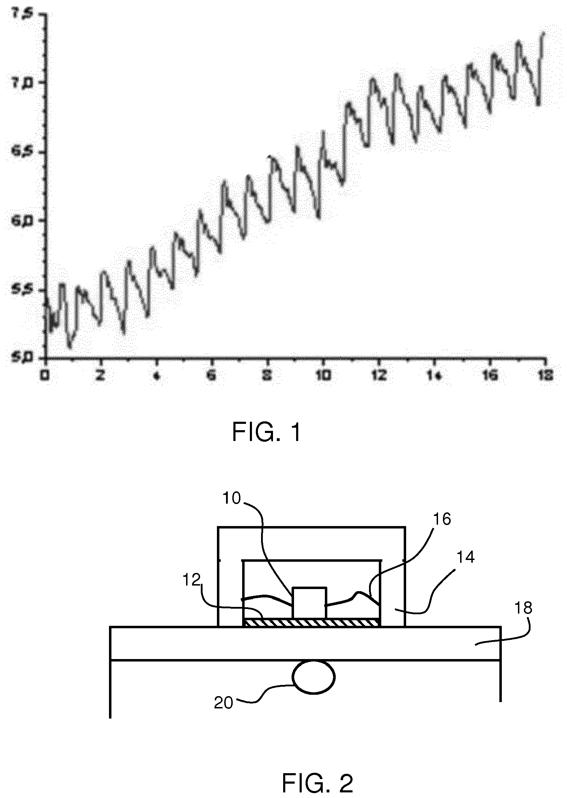

Figure 1 shows a graph of amplitude (mm) versus time (s) for a motion sensor, showing the skin doming to have a magnitude of the order of 0.5 mm. Figure 1 shows the skin doming superposed over a slower movement for example caused by general movement of the subject, which can be filtered out during signal processing.

Besides the weakness of the skin doming effect, there is also a location dependency. This location dependency needs to be taken into account for reliable and trustworthy pulse measurements. For example, the sensor typically needs to be placed above an artery, but only a rough estimation of an optimal placement can be achieved based on landmarks and/or via palpation. It would be desirable to be able to find the best location (and/or orientation) of a sensor targeting a specific signal source of interest such as the artery dilation and to minimize unwanted other signals, such as ballistocardiographic signals.

Thus, in summary, current sensor designs which are based on accelerometers have various problems which affect the measurement results. These include high inertial mass of the accelerometer, dampening of the measured effect, and no active and easy way to adjust the counter force of the sensor against the skin. Many examples suffer from low sensitivity. Some designs are also not able to distinguish clearly between vertical and lateral displacements.

A sensing solution for contact with the skin should thus have a low inertial mass, and have high sensitivity. The sensor should not be too sensitive to the location at which it is positioned and needs to be robust against motion artefacts (e.g. other body motions such as the head or neck) or facilitate easy detection of such artefacts. The sensor output should enable a reliable "Pulse" or "No Pulse" output to assist lay users.

SUMMARY OF THE INVENTION

The invention is defined by the claims.

According to examples in accordance with an aspect of the invention, there is provided a sensor apparatus for measuring a physiological parameter of a subject, wherein the sensor apparatus is adapted to be worn by the subject, comprising:

a housing;
a support structure carried by the housing, wherein the support structure has a contact portion which is adapted to be positioned by the housing against the skin of the subject;
a sensor for sensing movement of or pressure at the contact portion of the support structure; and
a positioning system for adjusting the in-plane position of the support structure and sensor relative to the housing.

This sensor design makes use of a support structure to decouple the sensor from the housing, so that there is a low inertial mass of the sensor, and hence a high sensitivity. In addition, a positioning system enables the signal to noise ratio to be maximize for a given placement of the housing, by enabling adjustment of the sensor position within the housing.

The sensor may be adapted to move relative to the housing, and then detect acceleration forces. Alternatively, it may comprise a pressure sensor for detecting pressure at the contact portion.

In this way, the user does not need to apply the sensor housing multiple times. Instead, there may be automatic position control or else outputs may be provided to the user to enable manual position adjustment but with the housing retaining a fixed position.

The system may use a single sensor (such as a single accelerometer or set of accelerometers) in order to enable optimized signal to noise ratio, by actively adjusting or guiding manual adjustment of the location of the sensor above an artery. This leads to improved robustness and performance of pulse detection in terms of sensitivity and specificity. It also enables improved workflow, since efforts in finding an optimal location (e.g. by palpation) either by the subject himself or by medical staff is reduced. Improved sensor calibration is also made possible due to a repeatable positioning and therefore sensitivity of the sensor.

The positioning system may provide position adjustment along one axis. If this axis is across the artery path, this single axis adjustment may be sufficient to obtain a strong signal. The positioning system may however allow position adjustment in two axis directions.

In one example, the support structure comprises a flexible membrane. This provides a low inertial mass, allowing the sensor to move freely relative to the housing.

In another example, the support structure comprises an air cushion. In this case, there may further be a system for adjusting the pressure of the air cushion. This provides a way to adjust the sensitivity of the sensor.

In another example, the support structure comprises a flexible material in which the sensor is embedded. There are thus various ways to implement the desired flexibility between the sensor and housing to provide decoupling between them.

In all examples, the sensor may comprise an accelerometer or a pressure sensor. They are both for monitoring movement. Multiple sensor types may also be used.

A further sensor may be provided for detecting movement of the housing. In this case, motion artefacts can be cancelled. This movement of the housing for example includes general motions of the human body (as well as movement of the housing relative to the body), and such movements can influence the measurements. Thus more accurate results are obtained by taking these movements into account.

In one example, the positioning system comprises a set of pulling members for pulling the support structure, wherein the pulling members comprise pulling lines or pulling webs. These may be used to pull the sensor to a desired position within the housing (in particular within a plane at the base of the housing).

In another example, the positioning system comprises a set of rollers for rolling and unrolling the support structure. The support structure may be rolled from one side and unrolled from the other side to effect a lateral shift.

In another example, the positioning system comprises a rotation device for rotating the support structure and wherein the sensor is off-center with respect to the support structure. In this way, rotation of the support structure effects a change in position of the sensor.

The sensor apparatus may further comprise a controller which is adapted to control the positioning system to position the sensor to achieve an optimized signal to noise ratio. This provides an automated adjustment routine. As an alternative, the controller may provide output signals which guide the user to provide the required position adjustment manually.

The housing for example comprises a patch for wearing against the skin, for example against the carotid artery. The sensor apparatus is preferably for measuring heart rate and/or respiration rate and/or heart rate variability and/or heart rhythm.

Examples in accordance with another aspect of the invention provide a method of measuring a physiological parameter of a subject, comprising:

applying a sensor apparatus against the skin of the subject, the sensor apparatus comprising a housing, a support structure carried by the housing with a contact portion which is positioned by the housing against the skin of the subject and a sensor for sensing movement of the contact portion of the support structure;
receiving sensor signals; and
adjusting the in-plane position of the support structure and sensor relative to the housing to maximize the signal to noise ratio of the received sensor signals.

BRIEF DESCRIPTION OF THE DRAWINGS

Examples of the invention will now be described in detail with reference to the accompanying drawings, in which:

Figure 1 shows a graph of amplitude versus time for a motion sensor;
Figure 2 shows a first example of sensor apparatus;
Figure 3 shows how the stiffness influences the sensitivity;
Figure 4 shows a second example of sensor apparatus;
Figure 5 shows a third example of sensor apparatus;
Figure 6 shows a modification to Figure 5 which shows how a variable pressure for the air cushion may be implemented;

Figure 7 shows a fourth example of sensor apparatus in which the sensor is embedded in a soft membrane;
Figure 8 shows a fifth example of sensor apparatus which combines an air cushion, a pressure sensor, an EAP actuator and a second motion sensor;
Figure 9 shows an algorithm to improve the positioning of the sensor to detect the heartbeat;
Figure 10 shows optimal placement of a sensor in the left image and non-optimal placement in the right image;
Figure 11 shows how the apparatus may be controlled to provide automatic position adjustment;
Figure 12 shows a first implementation of the positioning system;
Figure 13 shows a second implementation of the positioning system;
Figure 14 shows a third implementation of the positioning system;
Figure 15 shows a fourth implementation of the positioning system; and
Figure 16 shows the overall system, in which a controller receives the sensor signals and generates an output signal.

DETAILED DESCRIPTION OF THE EMBODIMENTS

The invention provides a sensor apparatus which comprises a housing, a support structure carried by the housing and a sensor for sensing movement of a contact portion of the support structure. The sensor is adapted to move relative to the housing. The apparatus further comprises a positioning system for adjusting the in-plane position of the support structure and sensor relative to the housing. This sensor design makes use of a support structure to decouple the sensor from the housing, so that there is a low inertial mass of the sensor, and hence a high sensitivity. In addition, a positioning system enables the signal to noise ratio to be maximized for a given placement of the housing, by enabling adjustment of the sensor position within the housing.

A first aspect of the design relates to the sensitivity of the motion sensor and in particular is directed to the requirement of a low inertial mass, as well as the need to ensure an appropriate contact force to the skin area. This aspect is of particular interest for pulse detection with low damping effects and therefore high sensitivity. Some examples of this aspect also enable detection of the sensor contact to the skin and active adjustment of the counter force.

Various examples are shown below. Where the same components are present, the same reference numbers are used. Different features from different examples may also be combined.

Figure 2 shows a first example which aims primarily to reduce the inertial mass of the sensor configuration. The sensor 10, such as one or more accelerometer sensors, gyroscopes, pressure sensors or force-sensitive sensors such as strain gages or piezoelectric sensors, is attached to a thin and flexible support structure in the form of a membrane 12.

The support structure 12 is a carried by a rigid housing 14 which is attached to the skin. The electrical wiring to the sensor is realized by thin flexible wires or a flexible PCB connection 16. However, an alternative option is for the support structure to include conductor lines, for example formed as a conducting mesh.

The sensor 10 is thus able to freely move according to the force or pressure resulting from the skin doming applied to the support structure 12. The sensitivity may be tuned by adjusting the stiffness of the support structure.

Figure 2 shows the skin 18 and the artery 20 being monitored.

The rigid housing 14 is attached to the skin by an adhesive material. The support structure does not need to be adhesive, but the support structure could be covered with an adhesive layer as well.

This configuration gives a low inertial mass of the support structure, so enables high sensitivity and provides a highly integrated solution with low cost.

This design thus uses a thin and soft membrane to improve the mechanical coupling between the skin and the sensor.

This design has been tested for support structures with different rigidity (softness). For the test design, an accelerometer was glued using silicone onto a soft membrane. The electrical connections used thin electrical isolated copper wires (diameter 0.05 mm). Small surface mount device (SMD) capacitors (C= 15nF) at the electrical terminals were used as low-pass filters for the output signals of the accelerometer IC.

The stiffness variation was implemented by using the same soft membrane but with an attached stiff backing tape for one test case, and an additional printed circuit board for another test case, between the skin and sensor support structure.

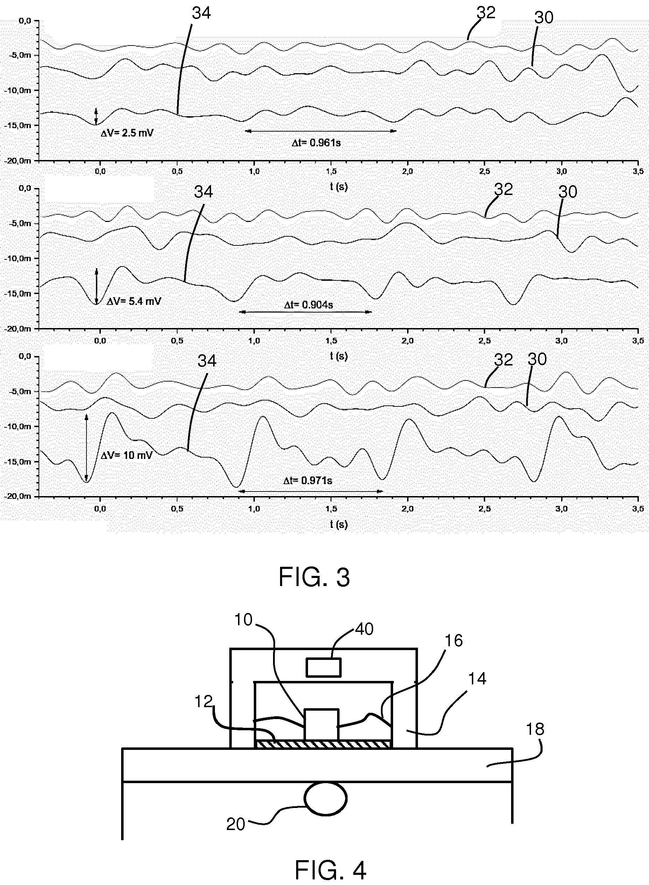

A comparison of the results is shown in Figure 3.

The top plot shows the sensor with rigid PCB, the middle plot shows the sensor with a backing tape and the bottom plot shows the sensor with only the soft membrane.

Each plot has three sub-graphs, each one showing Fast Fourier Transform (FFT) band-pass filtered (with filter frequencies of 0.5Hz and 5Hz) accelerometer data separated to the x-axis (plot 30), the y-axis (plot 32) and the z-axis (plot 34).

Since the skin doming caused by the pulse-wave is most dominant in the z-axis direction (perpendicular to the skin), the interpretation of the results may be limited to plot 34. In the sub-graphs the pulse width (At, representing the heart beat) and the peak-to-peak voltage (ΔV) of the z-accelerometer is shown.

Although the heart beat cycle can be determined in all three cases, a large difference in the sensing amplitude can be observed. Clearly the soft/flexible membrane shows the highest sensing signal, which is a factor of four higher than observed for the rigid housing configuration. This clearly shows the improved performance of an accelerometer sensor, if attached onto a soft/flexible support structure such as a soft membrane, which results in higher mechanical deflections and finally larger signal amplitudes with improved signal to noise ratio (SNR).

It is possible to dynamically control the stiffness of the membrane. This may be achieved with mechanical solutions. A preferred solution is however to use a thin electroactive polymer (EAP) actuator. As a function of the applied dc-operation voltage, the EAP deflects more or less and changes its stiffness at the same time. The EAP layer is for example double-clamped, i.e. clamped at opposite edges. Accordingly, the sensitivity to the skin doming can be adjusted and tuned to the user, such as the elasticity of the skin and the positioning of the device, or the applied pressure to the skin. This approach may be extended by using sensing as well as actuation functionality of the EAP layer, i.e. actuating for stiffness control and sensing for skin doming. Thus an EAP layer may provide an alternative or an additional sensing function to an accelerometer sensor.

Figure 4 shows a second example in which a second motion sensor 40 is attached to the housing 14 in order to reduce the sensitivity to motion artefacts. Ideally, the location of this sensor 40 is in close vicinity to the first sensor 10 but is not influenced by the skin doming caused by the heartbeat.

The sensor 40 may be implemented in the housing of the device (intentionally giving it a high inertial mass), so that only motion artefacts resulting from movements of the user are sensed. Since the sensor 10 detects both motions (motion artefacts and heart beat i.e. Ssignal + Snoise), such a configuration could be used to cancel the motion artefacts by subtraction:

$$\text{Sresult} = (\text{Ssignal} + \text{Snoise}) - \text{Snoise} = \text{Ssignal} \qquad (1)$$

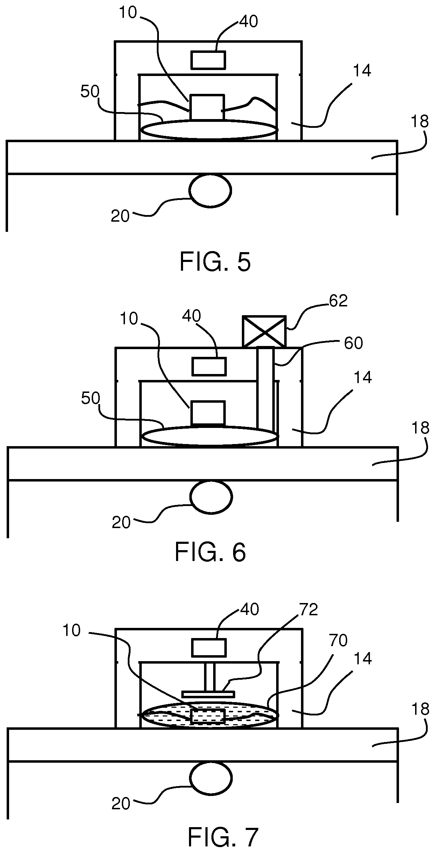

Figure 5 shows a third example in which the membrane 12 is replaced by a support structure in the form of an air cushion 50 in the form of an inflatable bladder. Depending on the internal pressure of the bladder, the sensitivity to the skin doming can be adjusted. Instead of air, also other compressible or non-compressible media may be filled in the bladder.

Special configurations, such as having a large bladder-skin interface but only a small bladder-sensor interface may be incorporated to "amplify" the movement of the sensor or the pressure/force applied to a pressure/force sensitive device.

Figure 6 shows a modification to Figure 5 which shows how a variable pressure for the air cushion 50 may be implemented. This allows the sensitivity of the configuration to be adapted by varying the pressure of the bladder. An external pressure control port 60 is provided which functions as an air inlet and outlet, controlled by a small pump and valve 62. The pressure control may be implemented manually and/or electronically, depending on user input or other sensors. The pressure may also be changed by means of other manual and/or electronic solutions, such as by compressing a closed bladder (to vary the internal pressure) or moving the bladder up or down.

The main sensor 10 may be inside the bladder, such as attached to the inner wall or detached from the bladder but mechanically coupled to the bladder by means of a rigid connection.

Figure 7 shows a fourth example in which the membrane 12 is replaced by a support structure in the form of soft membrane 70 in which the sensor 10 is embedded. The sensor 10 may be partly or fully embedded in the soft material or fluid, such as silicone, which is for example tuned to have the same mechanical properties (e.g. shore value) as the skin. In such a configuration, almost no scattering or reflection of the mechanical wave at the skin-silicone interface may be generated. On the opposite side of the sensor may be a rigid support 72, with an adjustable position in order to adapt the sensitivity.

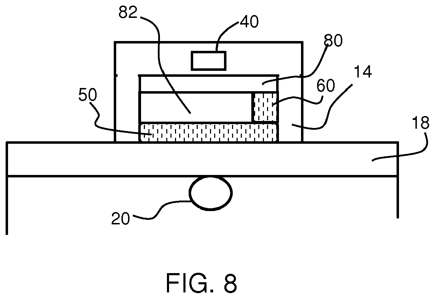

Figure 8 shows a fifth example which combines an air cushion 50, a pressure sensor 80, an EAP actuator 82 and a second motion sensor 40 fixed to the housing.

This combination of different sensor principles provides an adaptable solution giving flexibility and optimized performance. The second motion sensor 40 again is used to detect and reduce the influence of motion artefacts. The pressure sensor 80 is used to detect the skin doming caused by the heartbeat. Thus, the pressure sensor 80 functions as the main sensor for sensing a pressure at the contact portion of the support structure, transferred through the cushion, rather than sensing displacement directly.

Also, the pressure sensor 80 may perform a double function of sensing skin doming caused by the heart beat (as explained above) but alternatively it may be used to measure a pressure applied to the skin in addition, so that the heart beat based skin doming would be a superposed signal.

The manually and/or electronically operated air bladder 50 has a variable pressure and it is used to adapt the sensitivity to the heart beat detection. Finally, the electroactive polymer actuator 82 is used to fine tune the sensitivity and/or sensing purpose such as sensing the pressure of the heart beat or the pressure applied by the user to the skin or to provide a heartbeat detection signal.

The description above relates primarily to the decoupling of the sensor movement from the housing to enable high sensitivity. Another aspect relates to positioning of the sensor in the most suitable location for a high signal to noise ratio.

The sensor amplitude strongly depends on the position of the sensor on the skin, in relation to the artery, where the heart beat needs to be identified. If the sensor is far from the artery, the sensor output is much lower than if the sensor is positioned perpendicularly above the artery.

An algorithm may be used to improve the positioning of the sensor to detect the heartbeat.

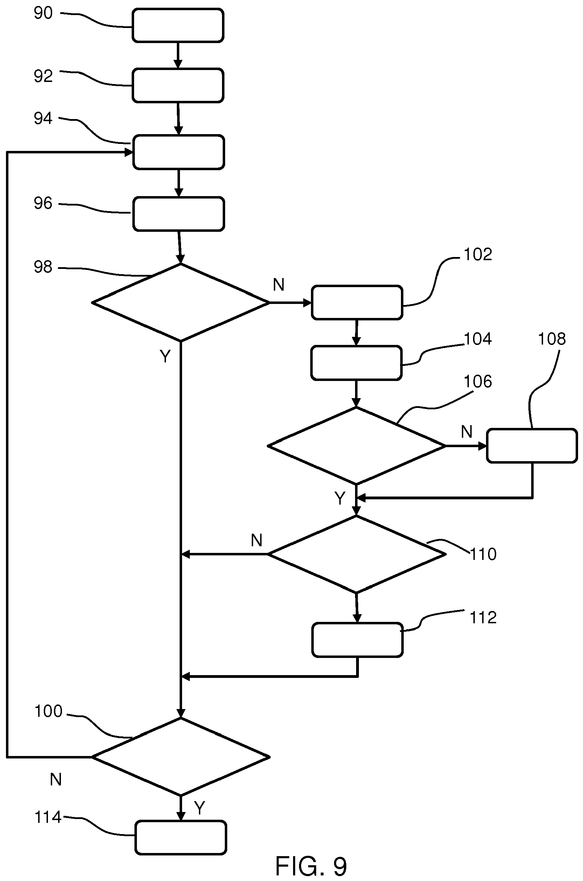

An example of a method to be implemented in software is shown in Figure 9.

The method starts in step 90.

An initial direction in which the sensor is to be moved is defined in step 92.

In step 94, a first sensor reading is taken, which may already be output to the user in step 96 via a display or a simple indicator.

As a first decision in step 98, it is checked if the sensor reading fulfills a certain precision and/or is within a given hysteresis or tolerance. For example, the signal to noise ratio (SNR) is taken as a reference. The SNR is defined as the relation of the signal-level to the noise-level:

$$\text{Signal to noise (SNR)} = P\_signal / P\_noise \quad (2)$$

P denotes the signal power.

If the SNR is high enough (i.e. the outcome is YES, Y), nothing else needs to be done except to check whether further sensing is required, for example based on a main on/off switch of the device. Thus, in step 100 it is determined when to stop sensing.

If the quality criterion is not reached (i.e. the outcome is NO, N), the sensor needs to be repositioned. This sequence starts with moving the sensor in the predetermined direction in step 102, then another sensor reading is made in step 104 followed by another decision in step 106 as to whether the second sensor reading is better or not.

If the sensor reading has not improved (i.e. the outcome is NO, N), the direction of movement is inverted in step 108. In both cases, of either an improved or a weakened SNR, an error check may follow in step 110 to check if anything went wrong and to guarantee non-endless loops. For example, it may be required to reposition the device manually because no position with sufficient SNR is found, or the device may need to be reset. This error handing takes place in step 112. If no error has been detected, or the error has been handled, the procedure can proceed.

Each cycle of the process thus provides one adjustment movement if it is needed. Once a suitable position has been found, the adjustment part of the loop is bypassed while the SNR remains high.

The process ends in step 114 when no further sensor readings are requested by the user for example when the device is turned off.

This method is for single direction adjustment of the position (backwards or forwards). Of course, full 2D position adjustment may also be employed. This method is simply an example to describe the basic procedure. Adaptations can easily be made in software code as known to experts skilled in the art.

Some position adjustment solutions will now be described.

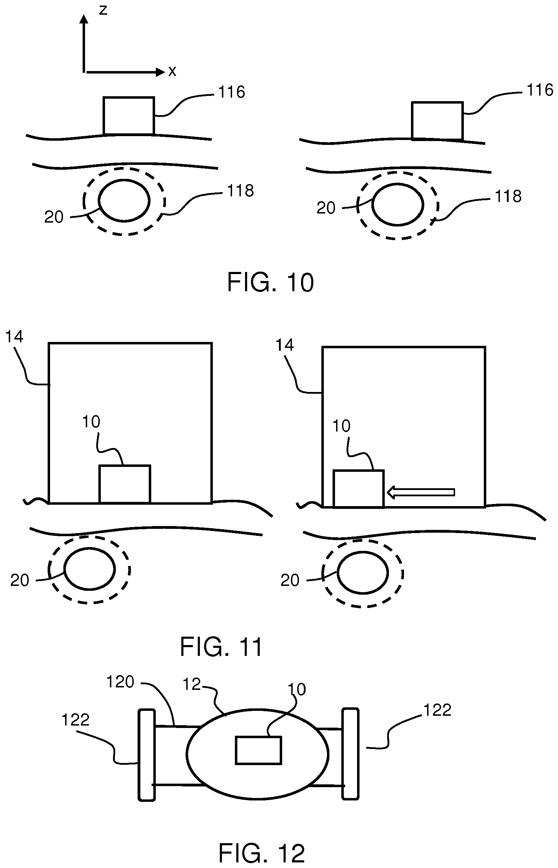

Figure 10 shows optimal placement in the left image and non-optimal placement in the right image. The sensor apparatus is shown generally as item 116. The pulsation of the artery and the resulting change in volume is indicated by the dashed lines 118. In an ideal situation, if the accelerometer is positioned perpendicularly above an artery, essentially the sensor apparatus would recognize only one direction, e.g. the z component (up-down) of the movement. The x-component (e.g. left-right) would be zero. The y-component may be non-zero, but this can be ignored. In particular, since the x-component is non-zero, it is clear that the sensor apparatus is not at an optimal position. Therefore, either the user needs to reposition the sensor apparatus by hand or an automatic tuning and repositioning mechanism needs to be used.

One approach is to provide an output to enable repositioning manually of the whole sensor apparatus 116. This may be supported by audible or visible indicators implemented in or on the device such as colored or flashing LEDs, or a line of LEDs or a loud speaker. When repositioning the sensor apparatus, the visible or audible signal varies such as by changing color from red (bad position) to green (good position), or an LED flashing frequency is increased with an improved position, or the number of illuminating LEDs increases as a function of the position. Similar approaches can be realized with audible signals.

This invention instead provides an improved solution whereby instead of requiring the whole sensor apparatus to be repositioned manually, adjustment of the sensor position within the overall apparatus may be made.

A basic approach is to enable manual readjustment of the sensor position within a static housing. The light or sound outputs described above may be used for this purpose. In this case, the step 102 of Figure 9 is carried out by the user following an appropriate output instruction.

A preferred approach is to provide automatic adjustment. In this case, the step 102 of Figure 9 is carried out automatically.

Figure 11 shows the basic approach.

The left image shows the sensor 10 at a first position within the housing 14 with a non-ideal location. The right image shows the sensor 10 moved to a better second location within the housing.

To maintain the low inertial mass, the support structure is moved with the sensor, rather than having a direct drive of the sensor itself. This maintains the desired decoupling.

Figure 12 shows a first implementation of the positioning system. The support structure 12 is attached to a set of pulling members 120 for pulling the support structure 12, wherein the pulling members comprise pulling lines. These may for example be small nylon strings. Each string can be rolled around a roller 122 which may then be electrically driven by miniature electric motors. Accordingly, the sensor can be repositioned according to the roller operation. If one roller is rolling up the opposite roller will be unrolling. Each string may be driven by a corresponding roller or strings can be rolled together on one roller per side. There may be position adjustment along one axis or along two orthogonal axes. The pulling members may also be on one side only by providing biasing means such as mechanical springs (to pull back the support structure) on the opposite side.

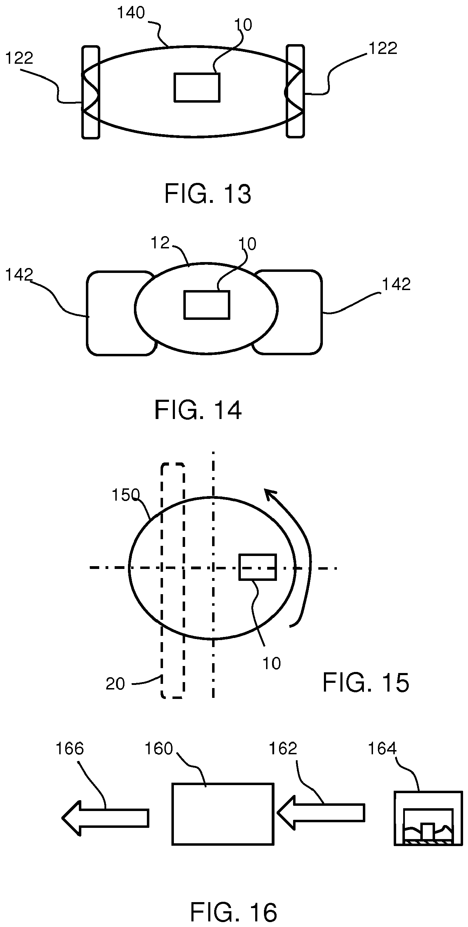

Figure 13 shows an alternative design based on a support structure in the form of a rollable belt 140. The belt can move the sensor 10 left and right. Opposite ends of the flexible support structure are again wrapped around rollers 122, which may be driven by miniature electronic motors.

Figure 14 shows an alternative design based on a support structure 12 being controlled by an electroactive polymer (EAP) actuator 142. These devices can be realized in many ways and in almost any form factor. Accordingly these components allow for several embodiments.

A first approach is to implement the pulling lines of Figure 12. There, the lines would be implemented by the EAP actuator, which can extend or shrink, depending on the applied voltage (voltage driven EAPs) or current (current driven ionic EAPs). Accordingly no rollers are then required. The EAP actuators are connected to the outer housing of the sensor apparatus. Further, a combination of pulling lines and EAPs, connected to each other may be used. In such a combined approach, a rough positioning may be realized by the pulling lines whereas a fine tuning may be realized by the EAP actuators. Finally instead of string-like EAP actuators, sheet-like EAP actuators at each side may be used as well.

Since EAPs can be made as thin (flexible) foils, the rolling belts such as in Figures 12 and 13 could also be used to roll up EAP foils (i.e. narrow foils to function as strings or a wide foil to function as a web) rolling around the ends of the rollers The motion of the rollers may be used to perform the raw positioning and the EAP actuator may be used for fine-tuning.

Thus, EAP actuators may be used to shift the sensor left and right by expanding or shrinking the EAP actuator. Alternatively, by rolling up actuator EAP foils around a roller, the sensor may be positioned the sensor to the desired place. The EAP actuation level may then be used to fine tune the positioning or to tune the rigidity of the EAP. The EAP functions as a membrane to decouple the sensor, so that a range or sensitivity setting may be realized by adjusting the rigidity.

The EAP actuator may be actuated even when partially rolled up. As long as the mechanical force or energy generated by the EAP actuator is not able to unroll the part of the rolled up EAP, the remaining part may be actuated.

The examples above are based on a linear position control.

An alternative is to provide a rotation device for rotating the support structure and wherein the sensor is off-center with respect to the support structure.

This approach is shown in Figure 15. The support structure comprises a disc 150. By rotating the disk 150 around its central (vertical) axis, the sensor can be repositioned until the sensor signal is maximized. As a simple drive scheme, the disc 150 may be driven by a transversal motor whose rotation axis is mechanically coupled to the disc. If the rotation axis is arranged centrally to the disk, a circular motion can be realized, whereas a non-central arrangement would result in an elliptical motion.

The sensor itself may be an accelerometer or other motion sensor. However, other sensors such as pressure, rotational, magnetic field or force sensors may be used but also optical solutions for example measuring a distance change caused by the palpitation of the heart and the consequent skin doming may be applicable as well.

As mentioned above, the basic principles may be used not only for position corrections along one axis, but also in a perpendicular direction.

Singe axis control is normally suitable if the device can be positioned so that it is known that the position adjustment is across rather than along the artery. However, full position control would enable the orientation of the sensor apparatus positioning to be arbitrary. This two directional positioning could also help to position the device at the best location when investigating arteries or vessels having a non-constant distance to the skin (e.g. deeper arteries or vessels).

Figure 16 shows the overall system, in which a controller 160 receives the sensor signals 162 from the sensor hardware 164 and generates an output signal 166.

The controller 160 may be within the housing of the device, and the device may then include an output device such as a display. Thus, the system may be fully self-contained. The controller 160 may instead be remote with wireless or wired communication from the sensor hardware 164 to the controller 160.

The controller 160 can perform various signal processing functions to improve the accuracy of the measurements and/or to minimize power consumption, and some of these approaches are discussed below.

For a sensor apparatus with multiple sensor types, for example an optical PPG sensor as well as a motion sensor, a first approach relates to the selection of which sensor modalities to use at any given time. If the signal to noise ratio of the motion sensor based pulse detection is high, the other sensing modalities (PPG or capacitive or bio-impedance) may then be switched off to save power. The output 166 is then derived from the motion sensor system only, which is a modality that is very low in power consumption. The other sensor modalities may be switched on as soon as the motion sensor system produces unreliable signals. Thus, additional sensors may be used when greater accuracy is desired.

Another indication of low quality signals may be when the pulse detection by one of the modalities (e.g. the motion-sensor based modality) differs from that of another modality (e.g. the PPG based modality).

In this way, one or more of the additional sensors may be activated in dependence on a signal quality associated with the motion sensor. The additional sensors may instead be operated periodically to make other measurements less frequently.

A second approach relates to use of the motion sensing to provide verification of the function of the other sensors. Accelerometer signals may for example be used to check if an optical sensor (e.g. PPG sensor) makes contact with the skin. If not, the user may be advised to reapply the patch.

In this way, an alert may be provided to the subject that sensor contact has been lost in dependence on the signal characteristics of the motion sensor. In particular, no local acceleration signals (i.e. no detected pulse signal) may be an indication that sensor contact has been broken.

A third approach relates to the combination of multiple sensor signals. An optical sensor system is sensitive to blood volume changes of the surface layers of skin, while the motion sensor system is sensitive to blood volume changes deep in the tissue.

The ratio of the two is indicative of the structure and response of the blood vessels. In addition, an average heart rate may be calculated as:

$$w*M1+(1-w)*M2$$

Here w is a weighting factor which may be determined based on the reliability of the first modality M1 (i.e. motion based), and the second modality M2 e.g. PPG, bio-impedance, or capacitive based. The reliability of M1 or M2 may be based on the level of distortion in the signals, in order to determine the weighting factor w.

When PPG-sensing is turned on, distortion of the signal by ambient light may be compensated by known techniques, such as using a duty cycle in which the LEDs are turned on and turned off and where the signal in the off-state is subtracted from the signal in the on- state. Angle selective filtering in front of the PPG photodetector or wavelength selective filtering may also be used (e.g. an infrared-blocking filter).

The invention is of primary interest for a wearable device for pulse detection to derive one or more of heart rate, heart rate variability and heart rhythm (e.g. detection of atrial fibrillation). Respiration rate may also be obtained. Such a wearable device can be used in a general hospital ward or at home.

Other possible measurements which may be made in addition to motion sensing include the blood oxygen saturation (SpO2) using an SpO2 sensor such as a PPG sensor, ECG measurements using ECG electrodes in contact with the skin, and ultrasound measurements using an ultrasound transducer. Thus, additional sensors may be provided within the housing, mounted on the support structure.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed examples can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

**Claims**

1. A sensor apparatus for measuring a physiological parameter of a subject, wherein the sensor apparatus is adapted to be worn by the subject, comprising:

   a housing (14);
   a support structure (12; 50; 70) carried by the housing, wherein the support structure has a contact portion which is adapted to be positioned by the housing against the skin of the subject;
   a sensor (10) for sensing movement of, or pressure at, the contact portion of the support structure; and
   a positioning system for adjusting the in-plane position of the support structure and sensor relative to the housing.

2. A sensor apparatus as claimed in claim 1, wherein the support structure comprises a flexible membrane (12).

3. A sensor apparatus as claimed in claim 1, wherein the support structure (12) comprises an air cushion (50).

4. A sensor apparatus as claimed in claim 3, further comprising a system (62) for adjusting the pressure of the air cushion.

5. A sensor apparatus as claimed in claim 1, wherein the support structure comprises a flexible material (70) in which the sensor is embedded.

6. A sensor apparatus as claimed in any preceding claim, wherein the sensor (10) comprises an accelerometer or a pressure sensor.

7. A sensor apparatus as claimed in any preceding claim, further comprising a further sensor (40) for detecting movement of the housing.

8. A sensor apparatus as claimed in any preceding claim, wherein the positioning system comprises a set of pulling members (120) for pulling the support structure, wherein the pulling members comprise pulling lines or pulling webs.

9. A sensor apparatus as claimed in any one of claims 1 to 8, wherein the positioning system comprises a set of rollers (122) for rolling and unrolling the support structure.

10. A sensor apparatus as claimed in any one of claims 1 to 8, wherein the positioning system comprises a rotation device for rotating the support structure (150) and wherein the sensor (10) is off-center with respect to the support structure.

11. A sensor apparatus as claimed in any preceding claim, further comprising a controller (160) which is adapted to control the positioning system to position the sensor to achieve an optimized signal to noise ratio.

12. A sensor apparatus as claimed in any preceding claim, wherein the housing comprises a patch for wearing against the skin, for example against the carotid artery.

13. A sensor apparatus as claimed in any preceding claim, for measuring heart rate and/or respiration rate and/or heart rate variability and/or heart rhythm.

14. A method of measuring a physiological parameter of a subject, comprising:

applying a sensor apparatus against the skin of the subject, the sensor apparatus comprising a housing (14), a support structure (12) carried by the housing with a contact portion which is positioned by the housing against the skin of the subject and a sensor (10) for sensing movement of the contact portion of the support structure; receiving sensor signals; and
adjusting the in-plane position of the support structure and sensor relative to the housing to maximize the signal to noise ratio of the received sensor signals.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

Europäisches
Patentamt

European
Patent Office

Office européen
des brevets

## EUROPEAN SEARCH REPORT

Application Number

EP 17 18 2481

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 269 312 A (KAWAMURA NORIO [JP] ET AL) 14 December 1993 (1993-12-14) * abstract; figures 1-4 * * column 1, line 50 - column 3, line 22 * * column 3, line 54 - column 7, line 54 * ----- | 1-14 | INV. A61B5/00 A61B5/024 A61B5/0205 ADD. A61B5/1455 |
| A | WO 2016/096391 A1 (KONINKL PHILIPS NV [NL]) 23 June 2016 (2016-06-23) * abstract; figures 1-10 * * page 12, line 15 - page 22, line 30 * ----- | 1-14 | |
| A | US 2004/010199 A1 (HASHIMOTO MASAO [JP] ET AL) 15 January 2004 (2004-01-15) * abstract; figures 1-25 * * paragraphs [0038] - [0099], [0110] - [0138] * ----- | 1-14 | |
| A | JP 2017 121406 A (OMRON HEALTHCARE CO LTD) 13 July 2017 (2017-07-13) * abstract; figures 1-7 * * paragraphs [0001] - [0035] * ----- | 1-14 | |

TECHNICAL FIELDS
SEARCHED       (IPC)

A61B

The present search report has been drawn up for all claims

| Place of search | Date of completion of the search | Examiner |
|---|---|---|
| The Hague | 17 January 2018 | Carta, Riccardo |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .............................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 18 2481

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2018

| Patent document cited in search report | | Publication date | Patent family member(s) | | Publication date |
|---|---|---|---|---|---|
| US 5269312 | A | 14-12-1993 | DE | 4224060 A1 | 04-02-1993 |
| | | | FR | 2679760 A1 | 05-02-1993 |
| | | | GB | 2258312 A | 03-02-1993 |
| | | | JP | H0511906 U | 19-02-1993 |
| | | | US | 5269312 A | 14-12-1993 |
| WO 2016096391 | A1 | 23-06-2016 | CN | 106999058 A | 01-08-2017 |
| | | | EP | 3232904 A1 | 25-10-2017 |
| | | | US | 2017347957 A1 | 07-12-2017 |
| | | | WO | 2016096391 A1 | 23-06-2016 |
| | | | WO | 2016097271 A2 | 23-06-2016 |
| US 2004010199 | A1 | 15-01-2004 | AU | 2003203909 A1 | 27-11-2003 |
| | | | CN | 1456125 A | 19-11-2003 |
| | | | DE | 60318254 T2 | 11-12-2008 |
| | | | EP | 1360930 A1 | 12-11-2003 |
| | | | EP | 1598004 A1 | 23-11-2005 |
| | | | JP | 3972141 B2 | 05-09-2007 |
| | | | JP | 2003325463 A | 18-11-2003 |
| | | | US | 2004010199 A1 | 15-01-2004 |
| JP 2017121406 | A | 13-07-2017 | JP | 2017121406 A | 13-07-2017 |
| | | | WO | 2017119185 A1 | 13-07-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Biowatch: Estimation of heart and breathing rates from wrist motions. **J. HERNANDEZ ; D. MCDUFF ; R.W. PICARD.** 9th International Conference on Pervasive Computing Technologies for Healthcare. PervasiveHealth, 2015, 169-176